# EUROPÄISCHE PATENTANMELDUNG

(11) **EP 3 742 758 A1**
(43) Veröffentlichungstag der Anmeldung: **25.11.2020**
(21) Anmeldenummer: 20173500.8
(22) Anmeldetag: 07.05.2020
(51) Int. Cl.: H04R 25/00, H04R 1/10, A61B 5/0245, A61B 5/024, A61B 5/044, A61B 5/0408, A61B 5/00

(54) **HÖRSYSTEM MIT EINEM HÖRINSTRUMENT**

(30) Priorität: 20.05.2019 DE 102019207373
(71) Anmelder: Sivantos Pte. Ltd., Singapore 539775 (SG)
(72) Erfinder: HUSUNG, Kunibert, 91052 Erlangen (DE)
(74) Vertreter: FDST Patentanwälte

(57) **Zusammenfassung**

Die Erfindung nennt ein Hörsystem (1), umfassend ein Hörinstrument (2) mit einer ersten Sensorelektrode (20) und einer zweiten Sensorelektrode (22) und eine Auswerteeinheit (34), wobei die Auswerteeinheit (34) dazu eingerichtet ist, anhand eines von der ersten Sensorelektrode (20) ausgegebenen ersten Sensorsignals (30) und eines von der zweiten Sensorelektrode (22) ausgegebenen zweiten Sensorsignals (32) ein Elektrokardiogramm (29) eines Trägers (54) des Hörinstrumentes (2) zu erstellen, wenn das Hörinstrument (2) vom Träger (54) bestimmungsgemäß getragen wird, und die erste Sensorelektrode (20) sowie die zweite Sensorelektrode (22) jeweils in Kontakt mit einem Körperteil (56) des Trägers (54) stehen. Die Erfindung nennt weiter ein entsprechendes Hörinstrument (2).

## Beschreibung

Die Erfindung betrifft ein Hörsystem mit einem Hörinstrument und einer Auswerteeinheit.

In einem Hörgerät wird durch einen elektro-akustischen Eingangswandler, welcher meist durch ein Mikrofon gegeben ist, ein Umgebungsschall in ein entsprechendes elektrisches Signal umgewandelt, welches anschließend in Abhängigkeit einer zu korrigierenden Hörschwäche des Trägers des Hörgerätes verarbeitet wird und dabei insbesondere frequenzbandabhängig verstärkt wird. Das aus der Signalverarbeitung resultierende Ausgangssignal wird von einem Schallerzeuger des Hörgerätes, welcher in Form eines Lautsprechers oder auch eines Knochenleitungshörers vorliegen kann, dem Gehör des Trägers zugeführt. Bei der Signalverarbeitung können zudem auch eine Rauschunterdrückung, eine Unterdrückung von akustischer Rückkopplung, ein Hervorheben bestimmter Schallquellen wie z.B. eines Gesprächspartners mittels Richtmikrofonie oder sonstige Prozesse zur Verbesserung der Qualität des Ausgangssignals, insbesondere hinsichtlich seines Nutzsignalanteils, durchgeführt werden.

Hörschwächen, welche bei einem Träger mittels eines Hörgerätes korrigiert werden sollen, treten insbesondere bei älteren Menschen auf, welche oftmals einen altersbedingten Hörverlust gerade in höheren Frequenzbändern zeigen, wodurch bei den betroffenen Personen infolge des natürlichen Frequenzspektrums von Sprache das akustische Sprachverständnis, also die Fähigkeit einer akustischen Auflösung von Sprache, beeinträchtigt wird. Ältere Menschen haben oftmals jedoch auch noch weitere medizinische Probleme, welche eine regelmäßige Überwachung erfordern können.

Auf der anderen Seite führt die zunehmende Miniaturisierung elektronischer Bauteile wie Sensoren, aber auch Bauteilen zur Datenübertragung, bereits jetzt zu einer Erweiterung des Funktionsumfangs von Hörinstrumenten wie Hörgeräten, aber auch Ohrhörern, welche von normal hörenden Personen z.B. zum Hören von Musik oder in Verbindung mit Mobiltelefonen eingesetzt werden. Insbesondere werden auch Hörgeräte drahtlos an externe Geräte wie Mobiltelefone gekoppelt, um dort beispielsweise ein bestimmtes Hörprogramm für eine konkrete Hörsituation anzuwählen, oder direkt Klangeinstellungen variieren zu können.

Der Erfindung liegt die Aufgabe zugrunde, ein Hörsystem mit einem Hörinstrument anzugeben, welches einen erweiterten Funktionsumfang aufweist, und dabei insbesondere dazu eingerichtet ist, medizinisch relevante Informationen zu erzeugen.

Die genannte Aufgabe wird erfindungsgemäß gelöst durch ein Hörsystem, umfassend ein Hörinstrument mit einer ersten Sensorelektrode und einer zweiten Sensorelektrode, und eine Auswerteeinheit, wobei die Auswerteeinheit dazu eingerichtet ist, anhand eines von der ersten Sensorelektrode ausgegebenen ersten Sensorsignals und eines von der zweiten Sensorelektrode ausgegebenen zweiten Sensorsignals ein Elektrokardiogramm (EKG) eines Trägers des Hörinstrumentes zu erstellen, wenn das Hörinstrument vom Träger bestimmungsgemäß getragen wird, und die erste Sensorelektrode sowie die zweite Sensorelektrode jeweils in Kontakt mit einem Körperteil des Trägers stehen. Vorteilhafte und teils für sich gesehen erfinderische Ausgestaltungen sind Gegenstand der Unteransprüche und der nachfolgenden Beschreibung.

Als Hörinstrument sind hierbei insbesondere ein Hörgerät zur Korrektur einer Hörschwäche eines Trägers des Hörgerätes sowie ein Ohrhörer zur Wiedergabe eines Audiosignals umfasst, wobei der Ohrhörer hierbei keine eigenständige Funktionen zur Korrektur von Hörschwächen aufzuweisen braucht.

Die Auswerteeinheit kann hierbei einerseits als Teil des Hörinstrumentes in selbiges integriert sein, also insbesondere innerhalb eines Gehäuses des Hörinstrumentes angeordnet sein, welches vom Träger an oder wenigstens teilweise in einem Ohr getragen wird. Im Fall eines Hörgerätes sind auch die wesentlichen Elemente der hörgerätespezifischen Signalerzeugung und -verarbeitung im Gehäuse integriert, also insbesondere mindestens ein elektro-akustischer Eingangswandler und eine mit dem Eingangswandler verbundene Signalverarbeitungseinheit. Bevorzugt ist im Fall einer in das Hörinstrument integrierten Auswerteeinheit diese jeweils mit der ersten Sensorelektrode und der zweiten Sensorelektrode verbunden.

Alternativ dazu kann die Auswerteeinheit Teil eines bezüglich des Hörinstrumentes externen Gerätes sein, also z.B. Teil einer Fernbedienung oder eines Mobiltelefons mit einer entsprechenden mobilen Applikation ("App"). In diesem Fall ist bevorzugt das Hörinstrument dazu eingerichtet, das erste Sensorsignal und das zweite Sensorsignal an das genannte externe Gerät, welches die Auswerteeinheit umfasst, zu übertragen, beispielweise mittels Radiofrequenz-Übertragung oder Bluetooth.

Die erste Sensorelektrode und die zweite Sensorelektrode sind hierbei ihrem Aufbau nachderart ausgestaltet, bei einem entsprechenden Kontakt mit einem Körperteil des Trägers des Hörinstrumentes ein erstes bzw. zweites Sensorsignal derart zu erzeugen, dass das erste Sensorsignal und das zweite Sensorsignal vom Informationsgehalt prinzipiell dazu geeignet sind, daraus ein EKG zu erstellen. Insbesondere sind die erste Sensorelektrode und die zweite Sensorelektrode dazu eingerichtet, eine elektrische Spannung oder Potentialdifferenz und/oder die Änderung einer solchen zu messen. Bevorzugt sind dabei die erste und die zweite Sensorelektrode auf der Oberfläche des Gehäuses des Hörinstrumentes angebracht, oder in selbige derart eingearbeitet, dass die beschriebene Messung einer Spannung an einem Körperteil, welches das Gehäuse an der entsprechenden Stelle der betreffenden Sensorelektrode berührt, technisch möglich ist, also die Sensorsignale eine für das Erstellen eines EKG ausreichende Signalstärke aufweisen.

Im Betrieb wird das Hörinstrument vom Träger bestimmungsgemäß am Ohr getragen, oder wenigstens teilweise in einen Gehörgang eingeführt, sodass durch einen Schallerzeuger des Hörinstrumentes ein vom Hörinstrument erzeugter Ausgangsschall dem Gehör des Trägers zugeführt werden kann. Das bestimmungsgemäß Tragen wird dabei insbesondere durch die Form des Hörinstrumentes sowie ggf. durch die Position seines Eingangswandlers bzw. seiner Eingangswandler und seines Schallerzeugers definiert. Insbesondere wird dabei wenigstens für eine Sensorelektrode bereits durch das bestimmungsgemäße Tragen, mit anderen Worten durch die Positionierung des Hörinstrumentes, der Kontakt mit einem Körperteil des Trägers hergestellt, beispielsweise mit der Haut am Eingang des Gehörgangs, an der Concha oder im Bereich der Ohrmuschel.

Das EKG kann dabei initiiert werden, indem der Träger aktiv den Kontakt eines Körperteils, beispielsweise eines Fingers, mit der zweiten Sensorelektrode herstellt. Der Beginn eines EKG kann aber auch über eine Anwahl einer entsprechenden Funktion am Hörinstrument selbst, oder in einer entsprechenden App o.ä. (im Fall eines Mobiltelefons als externem Gerät) erfolgen.

Bevorzugt ist die Anordnung der beiden Sensorelektroden im Hörinstrument derart, dass durch einen entsprechenden Kontakt mit Körperteilen und -stellen des Trägers über die Sensorelektroden ein elektrischer Kreislauf schließbar ist, welcher durch das Herz des Trägers führt, sodass das EKG-Signal besonders stark ausfällt, da es nicht von sekundären Spannungen im Körper abgeleitet zu werden braucht, sondern die elektrische Aktivität des Herzens direkt erfasst.

Bei einem Elektrokardiogramm werden elektrische Spannungsänderungen an der Körperoberfläche gemessen, welche durch elektrische Erregungen im Herzen hervorgerufen werden. Diese elektrischen Erregungen werden im sog. Erregungsbildungszentrum über das sog. Erregungsleitsystem des Herzens auf den Herzmuskel verteilt, um dessen Kontraktionen und somit die Pumpaktivität für den Blutkreislauf zu stimulieren. Im Ruhezustand weisen dabei Herzmuskelzellen i. Allg. ein negatives Membranpotential auf, d.h., der Extrazellularraum ist positiv geladen, während diese Relation bei elektrisch erregten Zellen umgekehrt ist. Die von einer sich einstellenden Ladungsverteilung im Extrazellularraum des Herzens hervorgerufene elektrische Spannung ist an der Haut messbar. Hierdurch lassen sich die periodischen Kontraktionen des Herzens über die ihnen jeweils vorausgehende und sie hervorrufende elektrische Aktivität am Herzen nachweisen und überprüfen. Insbesondere können dabei Abweichungen von den medizinisch üblichen Kontraktionsmustern erkannt werden.

Die vorliegende Erfindung erlaubt die Integration eines EKG in ein Hörinstrument, sodass gerade für Hörgeschädigte, welche überdies auch an gelegentlichen oder chronischen Problemen am Herzen leiden, wie dies insbesondere für ältere Personen häufiger der Fall ist, eine Überwachung der eigenen Herzaktivität auf besonders einfache Weise und ohne die Notwendigkeit zusätzlicher Geräte ermöglicht wird.

Bevorzugt ist die erste Sensorelektrode derart im Hörinstrument angeordnet, dass sie bei einem bestimmungsgemäßen Tragen frei zugänglich ist. Dies bedeutet im Fall, dass die erste Sensorelektrode nicht an der Oberfläche des Gehäuses liegt, sondern in dieses eingearbeitet ist, dass die entsprechende Stelle am Gehäuse frei zugänglich ist. Insbesondere hat dies zur Folge, dass bei einem bestimmungsgemäßen Tragen die Stelle im Gehäuse des Hörinstrumentes, an welcher die erste Sensorelektrode an der Oberfläche angeordnet ist oder in das Gehäuse eingearbeitet ist, vom Träger mit einem seiner Finger berührt werden kann, und ansonsten, ohne ein aktives Zutun des Trägers in Form einer Finger- und/oder Handbewegung, nicht in unmittelbarem Kontakt mit dem Körper des Trägers steht. Dies ermöglicht, eine EKG-Messung an einem bioelektrischen Stromkreis durchführen, welcher über den Arm des Trägers und somit am Herzen vorbei zum Kopf führt, wodurch die am Herzen entstehenden Potentialdifferenzen besonders gut messbar sind.

Günstigerweise ist dabei die Auswerteinheit dazu eingerichtet, das EKG des Trägers zu erstellen, wenn der Träger mit einem Teil einer Hand eine Stelle am Hörinstrument berührt, an welcher die erste Sensorelektrode angeordnet ist. Bevorzugt berührt hierbei der Träger das Hörinstrument mit einem Finger oder einem sonstigen Teil derselben Hand das Hörinstrument, auf welcher Seite auch das Hörinstrument getragen wird. Insbesondere wird das Hörinstrument am oder wenigstens teilweise im linken Ohr getragen, wobei der Träger für eine EKG-Messung die betreffende Stelle am Hörinstrument mit einem Finger oder sonstigen Teil der linken Hand berührt. Dies ist für den Träger eine einfach auszuführende Bewegung, durch welche ein vorteilhafter bioelektrischer Stromkreis für das EKG erreicht wird.

Zweckmäßigerweise umfasst das Hörsystem weiter eine Visualisierungseinrichtung, wobei die Auswerteeinheit dazu eingerichtet ist, ein erstelltes EKG an die Visualiserungseinrichtung zu übertragen, und wobei die Visualisierungseinrichtung dazu eingerichtet ist, ein empfangenes EKG graphisch darzustellen. Insbesondere kann die Visualisierungseinrichtung auch durch ein Mobiltelefon mit einer entsprechenden App zur Kommunikation mit dem Hörinstrument zwecks einer Übertragung des EKG sowie zur graphischen Darstellung desselben oder eine eigenständige, für das Hörinstrument dedizierte Fernbedienung implementiert sein. Bevorzugt führt der Träger das gesamte Hörsystem bei seiner bestimmungsgemäßen Verwendung, welche gegeben ist durch den bestimmungsgemäßen Gebrauch des Hörinstrumentes und die entsprechende Anwendung der Visualisierungseinrichtung, mit sich. Die Visualisierung eines EKG durch die genannte Visualisierungseinrichtung erlaubt dem Träger selbst eine erste Überprüfung des EKG auf mögliche Auffälligkeiten hin, ggf. auch individuell und spezifisch für seinen konkreten medizinischen Status.

Vorteilhafterweise ist dabei die Auswerteeinheit in die Visualisierungseinrichtung integriert. In diesem Fall werden insbesondere das erste Sensorsignal und das zweite Sensorsignal vom Hörinstrument, bevorzugt drahtlos, an die Visualisierungseinrichtung übertragen. Die Auswerteeinheit kann dabei insbesondere auch mittels einer Prozessoreinheit und einer Speichereinheit eines Mobiltelefons sowie einer entsprechenden App implementiert sein. Hierdurch lassen sich die rechnerischen Ressourcen der Visualisierungseinrichtung nutzen, ohne dass für ein EKG die Batterie des Hörinstrumentes über die Erstellung der Sensorsignale hinaus belastet wird. Eine Übertragung des EKG von der Auswerteeinrichtung an die Visualisierungseinrichtung erfolgt für den Fall, dass die Auswerteeinrichtung in die Visualisierungseinrichtung integriert ist, insbesondere über entsprechende Schnittstellen auf Hardware- und/oder Software-Ebene, sodass der für die Auswertung der Sensorsignale genutzte Bereich der Visualisierungseinrichtung das erstellte EKG an einen für die Visualisierung zuständigen Bereich ausgibt.

In einer weiter vorteilhaften, alternativen Ausgestaltung ist die Auswerteeinheit in das Hörinstrument integriert. Dies erlaubt es, lediglich eine fertige EKG-Kurve an die Visualisierungseinrichtung zur Darstellung zu übertragen, oder auch auf eine Visualisierung zu verzichten, und das EKG im Hörinstrument auf Auffälligkeiten zu überprüfen, wobei lediglich bei einer festgestellten Auffälligkeit eine Warnmaßnahme o.ä. erfolgt.

Als vorteilhaft erweist es sich, wenn das Hörinstrument als ein Ohrhörer ausgestaltet ist. Ein Ohrhörer wird von einem Träger zur Wiedergabe eines Audiosignals eingesetzt, wobei ein von einem Schallerzeuger des Ohrhörers erzeugter Ausgangsschall direkt in den Gehörgant eingeleitet wird. Der Ohrhörer weist dabei ggf. eine Signalverarbeitungseinheit auf, welche allg. zu einer frequenzbandabhängigen Verstärkung eines wiederzugebenden Eingangssignals eingerichtet sein kann, wobei diese Verstärkung vom Träger i.Allg. anhand dessen Hörpräferenzen ausgewählt werden kann, und üblicherweise nicht für eine Korrektur einer Hörschwäche des Trägers eingesetzt wird. Ohrhörer werden dabei im Zusammenhang mit Kommunikationsgeräten wie Mobiltelefonen, Tablet-PCs und dergleichen oder mit Unterhaltungselektronik wie z.B. mp3-Wiedergabegeräten eingesetzt. Da Ohrhörer oftmals auch beim Sport o.ä. getragen werden, kann ein Träger auf besonders einfache Weise ein EKG während einer Trainingsphase oder -pause erstellen lassen.

Als weiter vorteilhaft erweist es sich, wenn das Hörinstrument des Hörsystems als ein Hörgerät ausgestaltet ist, welches dazu vorgesehen und eingerichtet ist, eine Hörschwäche eines Trägers zu korrigieren. Hierdurch wird bei der Verwendung des Hörgerätes eine weitere, für die Gesundheit des Trägers wichtige und potentiell lebensrettende Funktion bereitgestellt, was insbesondere angesichts der Tatsache, dass Hörgeräte häufig von älteren Menschen getragen werden, bedeutsam ist.

In einer vorteilhaften Ausgestaltung des Hörsystems umfasst das Hörinstrument weiter ein Ohrstück, wobei die zweite Sensorelektrode im Ohrstück, bevorzugt an einer Außenfläche, angeordnet ist. Der Begriff eines Ohrstücks umfasst hierbei insbesondere jedwede Komponente eines Hörinstrumentes, welche für den bestimmungsgemäßen Gebrauch wenigstens teilweise in den Gehörgang oder in die Concha einzusetzen ist, also z.B. für ein als Hörgerät ausgestaltetes Hörinstrument einen an einem Schallschlauch angeordneten Dome oder einen vergleichbares Ohrpassstück im Fall eines Behind-The-Ear-Gerätes (BTE), ein Hörerstück im Fall eines Receiver-In-the-Canal-Gerätes (RIC), oder ein entsprechendes Gehäuseteil eines In-The-Ear-Gerätes (ITE). Im Fall eines Ohrhörers als Hörinstrument wird das Ohrstück gebildet durch einen Teil des Gehäuses oder das ganze Gehäuse des Ohrhörers. Im Fall eines Schallschlauches eines RIC-Gerätes ist die zweite Sensorelektrode bevorzugt am Dome bzw. am Ohrpassstück angeordnet, wobei am Schallschlauch entlang eine erste Sensorleitung zum Übertragen des zweiten Sensorsignals an eine Auswerteeinheit oder eine Kommunikationseinheit im Gehäuse führt. Eine Anordnung der zweiten Sensorelektrode in einem Ohrstück führt zu einer hohen Stabilität gegen Verschiebungen, da diese durch die fixierende Wirkung des Gehörgangs bzw. der Concha wirksam unterbunden werden

Als vorteilhaft erweist es sich auch, wenn das als Hörgerät ausgestaltete Hörinstrument weiter ein Gehäuse umfasst, welches für ein Tragen hinter einer Ohrmuschel des Trägers eingerichtet ist, wobei die zweite Sensorelektrode im Gehäuse, bevorzugt beim Tragen der Haut zugewandt, angeordnet ist. Insbesondere kann das Hörgerät als ein BTE-Gerät oder als ein RIC-Gerät ausgestaltet sein. Für derartige Hörgeräte kann es je nach detaillierter Ausgestaltung eines betreffenden Ohrstückes günstiger sein, auf eine erste Sensorelektrode im Ohrstück und eine entsprechende erste Sensorleitung entlang dem Ohrstück, welches oftmals reversibel mit dem Gehäuse verbunden wird, zu verzichten, und stattdessen die zweite Sensorelektrode an dem Teil des Gehäuses anzuordnen, welcher im Betrieb hinter der Ohrmuschel zu tragen ist, und somit auch durch diese gegen Verschiebungen, die zu Messfehlern in der EKG-Messung führen könnten, stabilisiert wird. Unter einem Tragen hinter der Ohrmuschel ist hierbei insbesondere jedwede Anordnung zu verstehen, bei welcher wenigstens ein Teil des Gehäuses aus seitlicher Sicht auf das Ohr hinter der Ohrmuschel angeordnet ist.

Zweckmäßigerweise ist das Hörinstrument weiter zum Messen eines Pulses des Trägers eingerichtet, wobei die Auswerteeinheit dazu eingerichtet ist, anhand des gemessenen Pulses und des EKG einen Blutdruck zu bestimmen. Insbesondere wird hierfür der gemessene Puls des Trägers von einer für die Messung des Pulses vorgesehenen Messvorrichtung an die Auswerteeinheit übertragen, oder es werden Messdaten einer derartigen Messvorrichtung an die Auswerteeinheit übertragen, sodass die Auswertung der Pulsmessung in der Auswerteeinheit erfolgt, insbesondere in einem eigenständigen, für die Pulsmessung dedizierten Modul der Auswerteeinheit, welches mit einem für die EKG-Messung dedizierten Modul der Auswerteeinheit in Kommunikation steht. Das EKG misst eine Herzaktivität im Wesentlichen ohne nennenswerte Zeitverzögerung, während eine Pulsmessung eine zeitverzögerte Information zur Herzfrequenz liefert, wobei die Laufzeitdifferenz zum in der EKG-Messung aufgelösten elektrischen Impuls am Herzen hierbei im Wesentlichen vom Blutdruck und ggf. vom Zustand der Blutgefäße abhängt. Bei Kenntnis des Zustandes der Blutgefäße können somit weitere hierdurch bedingte Verzögerungen der Laufzeit durch entsprechende Korrekturterme in den Rechnungen berücksichtigt werden, sodass durch das Hörsystem zusätzlich eine Blutdruckmessung möglich ist.

Bevorzugt weist dabei das Hörinstrument des Hörsystems zum Messen des Pulses des Trägers einen optischen Sensor auf. Zweckmäßigerweise umfasst der optische Sensor zumindest eine Lichtquelle und eine Detektionseinheit. Als Lichtquelle ist vorzugsweise eine LED eingesetzt, welche besonders bevorzugt derart im Hörinstrument angeordnet ist, dass bei einem bestimmungsgemäßen Tragen eine Emission von Licht in die Haut des Gehörgangs möglich ist. Das von der LED emittierte Licht wird von der Detektionseinheit, die vorzugsweise als eine Photodiode ausgebildet ist, detektiert. Das von dem optischen Sensor detektierte Signal ist bevorzugt ein Spannungs- oder Stromsignal. Zweckmäßigerweise ist dem Sensor ein Filter nachgeschaltet, der dazu dient, ein Signal des optischen Sensors zur Trennung von Rausch-, Stör- oder Untergrundsignalen vorzufiltern, bevor das Signal der Pulsmessung der Auswerteeinheit zugeführt wird.

Bevorzugt umfasst das Hörsystem weiter eine Kommunikationseinheit, wobei die Auswerteeinheit dazu eingerichtet ist, auf ein medizinisch auffälliges Elektrokardiogramm hin eine erste Warninformation an die Kommunikationseinheit auszugeben, und wobei die Kommunikationseinheit dazu eingerichtet ist, auf Basis der ersten Warninformation hin ein Notfallsignal an ein medizinisches Personal zu übermitteln. Die Kommunikationseinheit kann hierbei beispielsweise mittels eines Mobiltelefons und einer entsprechenden App zum Versenden des Notfallsignals über 3/4/5G o.ä. sowie ggf. zum Empfang der ersten Warninformation von einer im Hörinstrument angeordneten Auswerteeinheit implementiert sein. Ist die Auswerteeinheit selbst auch durch das Mobiltelefon implementiert, empfiehlt sich bevorzugt eine einzelne App, welche sämtliche Funktionen für das EKG sowie für die Übermittlung des Notfallsignals sowie ggf. für die Visualisierung des EKG bereitstellt. Je nach Unabhängigkeit des Trägers des Hörinstrumentes, aber auch nach einer festgestellten Schwere der Auffälligkeit des EKG ist der Träger möglicherweise nicht oder nicht mehr in der Lage, sich selbst zu helfen. In diesem Fall kann eine Übermittlung des Notfallsignals, welches bevorzugt zusätzlich zu medizinischen Daten wie dem EKG-Status auch eine ggf. codierte Patienten-Identifikation sowie GPS- oder ähnliche Lokalisierungsdaten umfasst, für den Träger lebensrettend sein.

Unter einem medizinisch auffälligen EKG ist hierbei und im Folgenden insbesondere ein EKG zu verstehen, welches generell oder auch für den Träger des Hörinstrumentes individuell und spezifisch betrachtet nachweisliche, maßgebliche Abweichungen aufweist, welche insbesondere einen gesundheitlichen Bedrohungszustand wiedergeben, oder zu einer gesundheitlichen Gefährdung führen können. Wird ein derartiges medizinisch auffälliges EKG von der Auswerteeinheit registriert, bevorzugt anhand eines Vergleiches mit einer Anzahl von in der Auswerteeinheit hinterlegten Referenz-EKGs, so wird von der Auswerteeinheit die erste Warninformation an die Signalverarbeitungseinheit ausgegeben. Ist dabei die Auswerteeinheit in einer Fernbedienung oder einem Mobiltelefon implementiert, so wird die Warninformation drahtlos an das Hörgerät übermittelt, und von einer oder der Signalverarbeitungseinheit entsprechend verarbeitet. Diese fügt nun in das Ausgangssignal, welches die Basis für den dem Gehör des Trägers zugeführten Ausgangsschall ist, das Warnsignal hinzu, sodass der Träger anhand des entsprechenden Warnsignaltons über die Auffälligkeit des EKG informiert wird, und ggf. einen Arzt aufsuchen kann oder ein Medikament zur Stabilisierung der Herztätigkeit einnehmen kann.

Zweckmäßigerweise weist das Hörinstrument wenigstens einen Schallerzeuger und eine Signalverarbeitungseinheit auf, wobei die Auswerteeinheit dazu eingerichtet ist, auf ein medizinisch auffälliges EKG hin eine zweite Warninformation an die Signalverarbeitungseinheit auszugeben, und wobei die Signalverarbeitungseinheit dazu eingerichtet ist, auf Basis der zweiten Warninformation ein Ausgangssignal, welches ein Warnsignal beinhaltet, an den wenigstens einen Schallerzeuger auszugeben. Der wenigstens eine Schallerzeuger kann hierbei insbesondere als ein Lautsprecher oder als ein Knochenleithörer ausgestaltet sein. Das Ausgangssignal wird vom Schallerzeuger in einen Ausgangsschall umgewandelt, welcher dem Gehör des Trägers zugeführt wird. Das Warnsignal kann hierbei zu einem Warnsignalton im Ausgangsschall, z.B. in Form eines markanten Pieptons oder -tonmusters, umgewandelt werden. Die zweite Warninformation kann dabei an die Signalverarbeitungseinheit auch unabhängig davon ausgegeben werden, ob eine erste Warninformation an die oder eine Kommunikationseinheit des Hörsystems ausgegeben wurde oder nicht.

Die Erfindung nennt weiter ein Hörinstrument mit einer ersten Sensorelektrode zur Erzeugung eines ersten Sensorsignals und einer zweiten Sensorelektrode zur Erzeugung eines zweiten Sensorsignals, wobei anhand des ersten Sensorsignals und des zweiten Sensorsignals ein Elektrokardiogramm eines Trägers des Hörinstrumentes erstellbar ist, wenn das Hörinstrument vom Träger bestimmungsgemäß getragen wird, und die erste Sensorelektrode sowie die zweite Sensorelektrode jeweils in Kontakt mit einem Körperteil des Trägers stehen. Das erfindungsgemäße Hörinstrument teilt die Vorteile des erfindungsgemäßen Hörsystems. Die Vorteile der Weiterbildungen des Hörsystems können dabei sinngemäß auf das Hörinstrument übertragen werden. Bevorzugt ist dabei das Hörinstrument als ein Hörgerät ausgestaltet.

Nachfolgend wird ein Ausführungsbeispiel der Erfindung anhand von Zeichnungen näher erläutert. Hierbei zeigen jeweils schematisch:
- Fig. 1: ein für eine EKG-Messung eingerichtetes Hörsystem mit einem BTE-Hörgerät und einem Mobiltelefon,
- Fig. 2: in einer Querschnittdarstellung ein für eine EKG-Messung eingerichtetes Hörsystem, welches in einem ITE-Hörgerät implementiert ist, und
- Fig. 3.: in einer Frontalansicht einen Träger eines Hörinstrumentes, welches durch einen Fingerdruck am Gehäuse zum Erstellen eines EKG befähigt wird.

Einander entsprechende Teile und Größen sind in allen Figuren jeweils mit denselben Bezugszeichen versehen.

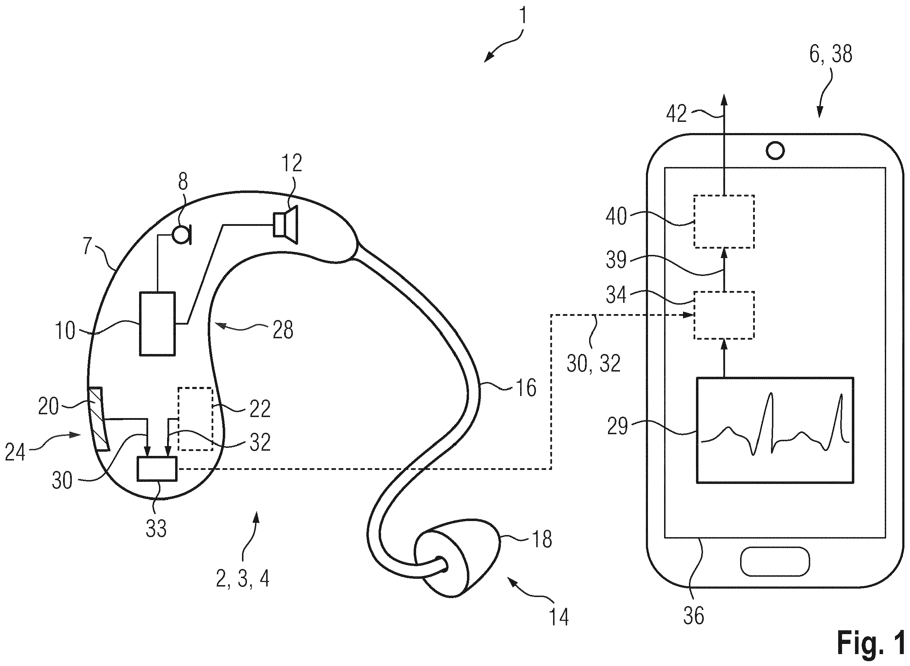

In Fig. 1 ist schematisch ein Hörsystem 1 mit einem Hörinstrument 2, welches hier als Hörgerät 3, konkret als ein BTE-Gerät 4 ausgestaltet ist, und einem Mobiltelefon 6 gezeigt, welche nicht maßstabsgetreu wiedergegeben sind. Das BTE-Gerät 2 weist in einem Gehäuse 7 einen ersten Eingangswandler 8 auf, welcher vorliegend als ein Mikrofon ausgestaltet ist, und zur Erzeugung eines elektrischen Eingangssignals aus einem Umgebungsschall eingerichtet ist. Der Eingangswandler 8 ist mit einer Signalverarbeitungseinheit 10 im Gehäuse 7 verbunden, welche das vom Eingangswandler 8 erzeugte Eingangssignal benutzerspezifisch, also insbesondere anhand der audiometrischen Anforderungen des Trägers des BTE-Gerätes 4, verarbeitet und dabei insbesondere frequenzbandabhängig verstärkt, um eine Hörschwäche des Trägers auszugleichen. Das Hörgerät 3 kann, insbesondere im Gehäuse 7, ggf. noch weitere in Fig. 1 nicht dargestellte Eingangswandler aufweisen, deren elektrische Eingangssignale ebenfalls der Signalverarbeitungseinheit 10 zu entsprechenden Verarbeitung zugeführt werden.

Die Signalverarbeitungseinheit 10 ist mit einem Schallerzeuger 12 verbunden, welcher vorliegend als ein Lautsprecher ausgestaltet ist, und ein fertig verarbeitetes Ausgangssignal der Signalverarbeitungseinheit 10 in einen Ausgangsschall umwandelt. Der Ausgangsschall wird im Betrieb des Hörgerätes 3 durch ein Ohrstück 14, welches einen Schallschlauch 16 und ein Ohrpassstück 18 umfasst, dem Gehör des Trägers zugeführt, indem das Ohrpassstück 18 derart in dessen Concha eingesetzt wird, dass der Auslass des Schallschlauchs 16 direkt an den Eingang seines Gehörgangs führt. Hierfür wird das Gehäuse 7 des BTE-Gerätes 4 im Betrieb vom Träger hinter dessen Ohrmuschel getragen.

Das BTE-Gerät 4 weist eine erste Sensorelektrode 20 und eine zweite Sensorelektrode 22 auf. Die erste Sensorelektrode 20 ist hierbei an Zarge 24 des Gehäuses 7, welche bei einem bestimmungsgemäßen Tragen des Gehäuses 7 hinter der Ohrmuschel dem Übergang von der Ohrmuschel zum Kopf abgewandt ist, derart angeordnet, dass sie nicht mit der Kopfhaut, der Ohrmuschel oder einem sonstigen Körperteil des Trägers in Berührung steht. Die zweite Sensorelektrode 22 ist in diejenige Seitenfläche 28 des Gehäuses 7 eingearbeitet, welche beim Tragen an der Kopfhaut des Trägers aufliegt. Diese Seitenfläche 28 sei in Fig. 1 die der Blickrichtung abgewandte Seitenfläche des Gehäuses 7. Die zweite Sensorelektrode 22 kann vorteilhafterweise auch an einem Innenbogen an der Unterseite des Gehäuses 7 angebracht sein. Hierdurch kann die zweite Sensorelektrode 22 bei der Messung fr eine bessere Kontaktgabe an die Haut gedrückt werden. Eine alternative Ausgestaltung, bei welcher die zweite Sensorelektrode in die gegenüberliegende, beim Tragen des BTE-Gerätes 4 an der Ohrmuschel anliegende Seitenfläche des Gehäuses 7 (also in die der Blickrichtung zugewandte Seitenfläche), eingearbeitet ist, kann ebenso realisiert werden.

Mittels der ersten Sensorelektrode 20 und der zweiten Sensorelektrode 22 kann vom Träger das Hörsystem 1 dazu verwendet werden, ein EKG 29 zu erstellen, und sich entsprechend anzeigen zu lassen. Hierfür wird durch die erste Sensorelektrode 20 ein erstes Sensorsignal 30 erzeugt, wenn der Träger die Stelle, an welcher die erste Sensorelektrode 20 im oder am Gehäuse 7 angeordnet ist, mit einem Teil der Hand berührt, bevorzugt mit einer Finderkuppe oder einem Fingerknöchel o.ä. Durch die zweite Sensorelektrode 22 wird entsprechend ein zweites Sensorsignal 32 erzeugt.

Die erste Sensorelektrode 20 und die zweite Sensorelektrode 22 können dabei an der äußeren Oberfläche des Gehäuses 7 angeordnet sein, oder in das Material des Gehäuses 7, bevorzugt nahe der äußeren Oberfläche, derart eingearbeitet sein, dass die entsprechenden, für das EKG 29 erforderlichen elektrischen Messungen möglich sind. Die zweite Sensorelektrode 22 kann in einer nicht näher dargestellten Alternative auch am Ohrpassstück 18 angeordnet sein, wobei in einem solchen Fall eine Sensorleitung am Schallschlauch 16 entlang verläuft, um das zweite Sensorsignal zum Gehäuse 7 zu führen.

Für das Erzeugen des ersten Sensorsignals 30 bzw. des zweiten Sensorsignals 32 kann für die erste Sensorelektrode 20 bzw. die zweite Sensorelektrode 22 im BTE-Gerät 4 eine nicht näher dargestellte Versorgung mit einer Betriebsspannung vorgesehen und eingerichtet sein. Insbesondere kann die erste Sensorelektrode 20 dabei auch eingerichtet sein, einen Kontakt mit einem Teil der Hand des Trägers zu registrieren, sodass eine Messung der elektrischen Aktivität am Herzen des Trägers zum Erstellen eines EKG 29 erst auf einen derartigen Kontakt hin initialisiert wird.

Für das Erstellen des EKG 29 werden das erste Sensorsignal 30 und das zweite Sensorsignal 32 auf geeignete, nicht näher beschriebene Weise durch ein Übertragungsmodul 33 an das Mobiltelefon 6 übertragen, beispielsweise mittels Bluetooth oder Nearfield-Communication oder dergleichen. Das Mobiltelefon 6 ist dabei für einen Empfang der beiden Sensorsignale 30, 32 über ein derartiges Kommunikationsprotokoll eingerichtet. Im Mobiltelefon 6 ist auf der internen Hardware mittels einer entsprechenden App eine Auswerteeinheit 34 implementiert, welche dazu eingerichtet ist, das erste Sensorsignal 30 und das zweite Sensorsignal 32 auszuwerten, und hieraus das EKG 29 zu erstellen.

Somit kann der Träger das aus dem Hörgerät 3 und dem Mobiltelefon 6 gebildete Hörsystem 1 dazu nutzen, wertvolle medizinische Information in Form des EKG 29 sofort selbst zu erhalten, wofür er lediglich die Messung und somit die Erzeugung der Sensorsignale 30, 32 initialisieren muss, und mit einem Finger o.ä. die Stelle der ersten Sensorelektrode 20 am Gehäuse 7 zu berühren braucht, um einen vorteilhaften elektrischen Stromkreis am Körper zu erzielen. Die Initialisierung des EKG 29 kann auch durch einen entsprechenden Befehl in der App erfolgen.

Weiter bildet das Mobiltelefon 6 eine Visualisierungseinrichtung 38 für das EKG 29. Hierfür ist die besagte App dazu konfiguriert, über einen Bildschirm 36 des Mobiltelefons 6 das von der Auswerteeinheit 34 erstellte und bereitgestellte EKG 29 graphisch darzustellen, sodass der Träger auffällige Werte sofort erkennen kann. Besonders auffällige oder gar für die Gesundheit des Trägers bedrohliche Werte des EKG 29 kann die mittels der App implementierte Auswerteeinheit 34 selbständig erkennen. In diesem Fall wird von der Auswerteeinheit 34 eine erste Warninformation 39 an eine Kommunikationseinheit 40 des Mobiltelefons 6 ausgegeben, sodass die Kommunikationseinheit 40 ein Notfallsignal 42 aussendet, welches von einem Arzt, einem Krankenhaus, einem medizinischen Notdienst o.ä. empfangen werden kann, um dem Träger umgehend medizinische Hilfe zukommen lassen zu können.

Die Kommunikationseinheit 40 ist hierbei mittels der nativen, im Mobiltelefon 6 zur Signalübertragung an ein mobiles Netz oder ein mobiles Datennetz vorgesehenen Komponenten (z.B. Antennen für 3/4/5G-Kommunikation o.ä.) implementiert, wobei beispielsweise die erste Warninformation 39 als das Notfallsignal 42 von den besagten Komponenten versandt werden kann, d.h., die App erzeugt die erste Warninformation 39 bereits in einem Datenformat und mit sämtlichen Informationen, welche der Empfänger des Notfallsignals 42 für eine schnellstmögliche Behandlung des Trägers 42 benötigt, und die Kommunikationseinheit 40 übermittelt diese Informationen als das Notfallsignal 42. Andere Implementierungen sind denkbar. Bevorzugt umfasst das Notfallsignal 42 dabei auch GPS-Daten und/oder vergleichbare Informationen zur Lokalisierung des Trägers.

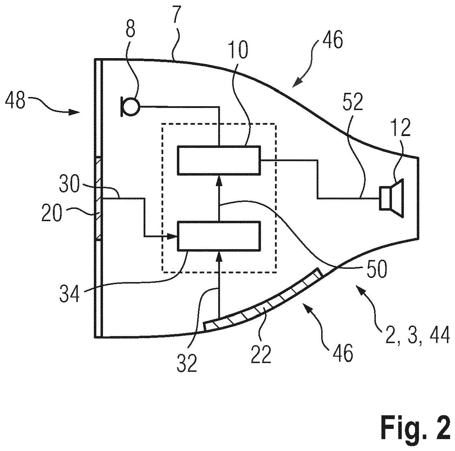

In Fig. 2 ist in einer Querschnittdarstellung ein Hörsystem 1 gezeigt, welches vollständig in einem als ITE-Gerät 44 ausgestalteten Hörgerät 3 implementiert ist. Das ITE-Gerät 44 wird für ein bestimmungsgemäßes Tragen teilweise in den Gehörgang des Trägers eingeführt. Hierfür weist das Gehäuse 7 eine dem äußeren Gehörgang nachempfundene, bevorzugt individuell auf die Anatomie des Trägers abgestimmte Ausformung 46 auf. Das Gehäuse des ITE-Gerätes 44 umfasst eine Abdeckplatte 48 (sog. "Faceplate"; senkrecht zur Blickrichtung), in welche die erste Sensorelektrode 20 derart integriert ist, dass diese beim Tragen des ITE-Gerätes 44 dem freien Raum neben dem Ohr zugewandt ist. Die zweite Sensorelektrode 22 ist in die Ausformung 46 am Gehäuse integriert. Die Auswerteeinheit 34 ist hierbei im Gehäuse 7 angeordnet, und kann dabei insbesondere auf demselben System-On-a-Chip 49 implementiert sein, auf welchem auch die Signalverarbeitungseinheit 10 implementiert ist.

Für ein EKG kann nun der Träger die Abdeckplatte 48 an der Stelle, über welche sich die die erste Sensorelektrode 20 erstreckt, mit einem Finger o.ä. berühren. Die zweite Sensorelektrode 22 ist, sobald das ITE-Gerät 44 getragen wird, ständig in Kontakt mit der Haut des Trägers. Die Auswertung des ersten Sensorsignals 30 und des zweiten Sensorsignals 32 erfolgt wie im anhand von Fig. 1 beschriebenen Ausführungsbeispiel in der Auswerteinheit 34, mit dem Unterschied, dass vorliegend die Auswerteeinheit im Gehäuse 7 angeordnet ist, und somit die Sensorsignale 30, 32 nicht an eine externe Einheit wie das Mobiltelefon 6 übertragen werden müssen. Die Visualisierung des erzeugten EKG kann analog zu Fig. 1 in einer externen Visualisierungseinrichtung (nicht dargestellt) erfolgen, welche in einem Mobiltelefon oder einer eigenen Fernbedienung des ITE-Gerätes 44 implementiert sein kann.

Im Fall eines medizinisch auffälligen EKG kann die Auswerteeinheit 34 eine zweite Warninformation 50 an die Signalverarbeitungseinheit 10 ausgeben - z.B. über eine entsprechende Schnittstelle - sodass in das an den Schallerzeuger ausgegebene Ausgangssignal 52 ein Warnsignal eingespeist wird, welches durch den Schallerzeuger 12 mit ausgegeben wird. Der Träger kann dann dieses Warnsignal hören, und wird somit über die Auffälligkeit informiert, sodass er umgehend einen Arzt, ein Krankenhaus, einen medizinischen Notdienst o.ä. aufsuchen kann.

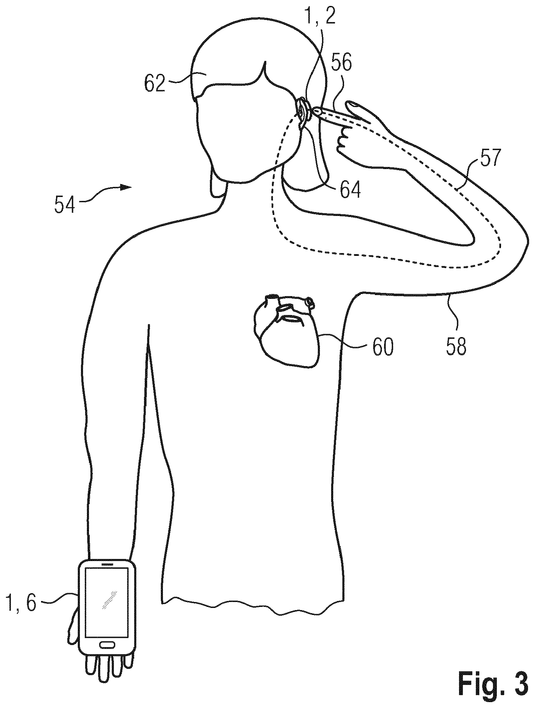

In Fig. 3 ist schematisch ein Träger 54 eines Hörinstrumentes 2 dargestellt. Das Hörinstrument 2 kann hierbei als ein Hörgerät, beispielsweise als das BTE-Gerät 4 nach Fig. 1 oder als das ITE-Gerät 44 nach Fig. 2 ausgestaltet sein, oder durch einen Ohrhörer gegeben sein. Das Hörinstrument 2 weist in nicht näher dargestellter Weise eine erste Sensorelektrode und eine zweite Sensorelektrode zum Erstellen eines EKG auf, und ist Teil des Hörsystems 1, welches überdies das Mobiltelefon 6 umfasst. Um mit dem Hörsystem 1 in der oben beschriebenen ein EKG zu erstellen, berührt der Träger 54 das Gehäuse des Hörinstrumentes 2 mit einem Finger 56 an derjenigen Stelle, an welcher die erste Sensorelektrode angeordnet ist. Hierdurch wird ein elektrischer Kreislauf 57 am Körper des Trägers 54 geschlossen, welcher über den Arm 58 am Herzen 60 vorbei zum Kopf 62 des Trägers und somit zum Ohr 64 führt, wo die zweite Sensorelektrode die Haut des Trägers berührt. Hierdurch wird ermöglicht, anhand der Sensorsignale der beiden Sensorelektroden die elektrische Aktivität des Herzens 60 ohne einen zu großen Einfluss anderer elektrischer Störsignale zu messen, und daraus ein KG zu erstellen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

### Bezugszeichenliste

- 1: Hörsystem
- 2: Hörinstrument
- 3: Hörgerät
- 4: BTE-Gerät
- 6: Mobiltelefon
- 7: Gehäuse
- 8: Eingangswandler
- 10: Signalverarbeitungseinheit
- 12: Schallerzeuger
- 14: Ohrstück
- 16: Schallschlauch
- 18: Ohrpassstück
- 20: erste Sensorelektrode
- 22: zweite Sensorelektrode
- 24: Zarge
- 28: Seitenfläche
- 29: EKG / Elektrokardiogramm
- 30: erstes Sensorsignal
- 32: zweites Sensorsignal
- 33: Übertragungsmodul
- 34: Auswerteeinheit
- 36: Bildschirm
- 38: Visualisierungseinrichtung
- 39: erste Warninformation
- 40: Kommunikationseinheit
- 42: Notfallsignal
- 44: ITE-Gerät
- 46: Ausformung
- 48: Abdeckplatte
- 49: System-On-a-Chip
- 50: zweite Warninformation
- 52: Ausgangssignal
- 54: Träger
- 56: Finger
- 57: elektrischer Kreislauf
- 58: Arm
- 60: Herz
- 62: Kopf
- 64: Ohr

## Patentansprüche

1. Hörsystem (1), umfassend ein Hörinstrument (2) mit einer ersten Sensorelektrode (20) und einer zweiten Sensorelektrode (22) und eine Auswerteeinheit (34),
wobei die Auswerteeinheit (34) dazu eingerichtet ist, anhand eines von der ersten Sensorelektrode (20) ausgegebenen ersten Sensorsignals (30) und eines von der zweiten Sensorelektrode (22) ausgegebenen zweiten Sensorsignals (32) ein Elektrokardiogramm (29) eines Trägers (54) des Hörinstruments (2) zu erstellen, wenn das Hörinstrument (2) vom Träger (54) bestimmungsgemäß getragen wird, und die erste Sensorelektrode (20) sowie die zweite Sensorelektrode (22) jeweils in Kontakt mit einem Körperteil (56) des Trägers (54) stehen.

2. Hörsystem (1) nach Anspruch 1,
wobei die erste Sensorelektrode (20) derart im Hörinstrument (2) angeordnet ist, dass sie bei einem bestimmungsgemäßen Tragen frei zugänglich ist.

3. Hörsystem (1) nach Anspruch 2,
wobei die Auswerteinheit (34) dazu eingerichtet ist, das Elektrokardiogramm (29) des Trägers (54) zu erstellen, wenn der Träger (54) mit Teil einer Hand (56) eine Stelle am Hörinstrument (2) berührt, an welcher die erste Sensorelektrode (20) angeordnet ist.

4. Hörsystem (1) nach einem der vorhergehenden Ansprüche, weiter umfassend eine Visualisierungseinrichtung (38),
wobei die Auswerteeinheit (34) dazu eingerichtet ist, ein erstelltes Elektrokardiogramm (29) an die Visualisierungseinrichtung (38) zu übertragen, und
wobei die Visualisierungseinrichtung (38) dazu eingerichtet ist, ein empfangenes Elektrokardiogram (29) graphisch darzustellen.

5. Hörsystem (1) nach Anspruch 4,
wobei die Auswerteeinheit (34) in die Visualisierungseinrichtung (38) integriert ist.

6. Hörsystem nach einem der vorhergehenden Ansprüche 1 bis 4,
wobei die Auswerteeinheit (34) in das Hörinstrument (2) integriert ist.

7. Hörsystem (1) nach einem der vorhergehenden Ansprüche,
wobei das Hörinstrument (2) als ein Ohrhörer ausgestaltet ist.

8. Hörsystem (1) nach einem der Ansprüche 1 bis 6,
wobei das Hörinstrument (2) als ein Hörgerät (3) ausgestaltet ist.

9. Hörsystem (1) nach einem der vorhergehenden Ansprüche, wobei das Hörinstrument (2) weiter ein Ohrstück (14) umfasst, und
wobei die zweite Sensorelektrode (22) im Ohrstück (14) angeordnet ist.

10. Hörsystem (1) nach Anspruch 8,
wobei das Hörgerät (2) weiter ein Gehäuse (7) umfasst, welches für ein Tragen hinter einer Ohrmuschel des Trägers (54) eingerichtet ist, und
wobei die zweite Sensorelektrode (22) im Gehäuse (7) angeordnet ist

11. Hörsystem (1) nach einem der vorhergehenden Ansprüche,
wobei das Hörinstrument (2) weiter zum Messen eines Pulses des Trägers (54) eingerichtet ist, und
wobei die Auswerteeinheit (34) dazu eingerichtet ist, anhand des gemessenen Pulses (54) und des EKG (29) einen Blutdruck zu bestimmen.

12. Hörsystem (1) nach Anspruch 11,
wobei das Hörinstrument (2) zum Messen des Pulses des Trägers (54) einen optischen Sensor aufweist.

13. Hörsystem (1) nach einem der vorhergehenden Ansprüche, weiter umfassend eine Kommunikationseinheit (40),
wobei die Auswerteeinheit (34) dazu eingerichtet ist, auf ein medizinisch auffälliges Elektrokardiogramm (29) hin eine erste Warninformation (39) an die Kommunikationseinheit (40) auszugeben, und
wobei die Kommunikationseinheit (40) dazu eingerichtet ist, auf Basis der ersten Warninformation (39) hin ein Notfallsignal (42) an ein medizinisches Personal zu übermitteln.

14. Hörsystem (1) nach einem der vorhergehenden Ansprüche,
wobei das Hörinstrument (2) wenigstens einen Schallerzeuger (12) und eine Signalverarbeitungseinheit (10) aufweist,
wobei die Auswerteeinheit (34) dazu eingerichtet ist, auf ein medizinisch auffälliges Elektrokardiogramm (29) hin eine zweite Warninformation (50) an die Signalverarbeitungseinheit (10) auszugeben, und
wobei die Signalverarbeitungseinheit (10) dazu eingerichtet ist, auf Basis der zweiten Warninformation (50) ein Ausgangssignal (52), welches ein Warnsignal beinhaltet, an den wenigstens einen Schallerzeuger (12) auszugeben.

15. Hörinstrument (2) mit einer ersten Sensorelektrode (20) zur Erzeugung eines ersten Sensorsignals (30) und einer zweiten Sensorelektrode (22) zur Erzeugung eines zweiten Sensorsignals (32), wobei anhand des ersten Sensorsignals (30) und des zweiten Sensorsignals (32) ein Elektrokardiogramm (29) eines Trägers (54) des Hörinstrumentes (2) erstellbar ist, wenn das Hörinstrument (2) vom Träger (54) bestimmungsgemäß getragen wird, und die erste Sensorelektrode (20) sowie die zweite Sensorelektrode (22) jeweils in Kontakt mit einem Körperteil (56) des Trägers (54) stehen.
